(19) Europäisches Patentamt
European Patent Office
Office européen des brevets

(11) Veröffentlichungsnummer: **0 295 482**
**A1**

(12) # EUROPÄISCHE PATENTANMELDUNG

(21) Anmeldenummer: 88108602.9

(51) Int. Cl.4: **C07D 231/44 , A01N 43/56**

(22) Anmeldetag: 30.05.88

(30) Priorität: **12.06.87 DE 3719733**

(43) Veröffentlichungstag der Anmeldung:
**21.12.88 Patentblatt 88/51**

(84) Benannte Vertragsstaaten:
**BE CH DE ES FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Jensen-Korte, Uta, Dr.**
**Geibelstrasse 9**
**D-4000 Düsseldorf 1(DE)**
Erfinder: **Schallner, Otto, Dr.**
**Noldeweg 22**
**D-4019 Monheim(DE)**
Erfinder: **Stetter, Jörg, Dr.**
**Gellertweg 4**
**D-5600 Wuppertal 1(DE)**
Erfinder: **Andrews, Peter, Dr.**
**Gellertweg 2**
**D-5600 Wuppertal 1(DE)**
Erfinder: **Becker, Benedikt, Dr.**
**Metzkausener Strasse 14**
**D-4020 Mettmann(DE)**
Erfinder: **Homeyer, Bernhard, Dr.**
**Obere Strasse 28**
**D-5090 Leverkusen 3(DE)**
Erfinder: **Stendel, Wilhelm, Dr.**
**In den Birken 55**
**D-5600 Wuppertal 1(DE)**
Erfinder: **Behrenz, Wolfgang, Dr.**
**Untergründemich 14**
**D-5063 Overath(DE)**

(54) **Substituierte 5-Ethylamino-1-arylpyrazole.**

(57) Es werden neue substituierte 5-Ethylamino-1-arylpyrazole der Formel

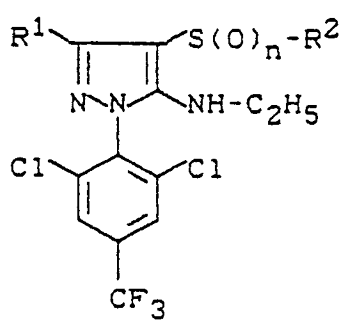

(I)

EP 0 295 482 A1

bereitgestellt,
in welcher
$R^1$ für Wasserstoff oder Alkyl steht,
$R^2$ für Halogenalkyl steht und
n für eine Zahl 0, 1 oder 2 steht,
mit Ausnahme der Verbindung der Formel (I), bei welcher $R^1$ für Wasserstoff, $R^2$ für Dichlorfluormethyl und gleichzeitig n für 0 steht.

Die Verbindungen der Formel (I) zeigen eine stark ausgeprägte Wirksamkeit gegen tierische Schädlinge, insbesondere gegen Insekten und Nematoden und sind hervorragend als Wirkstoffe in Schädlingsbekämpfungsmitteln geeignet.

## Substituierte 5-Ethylamino-1-arylpyrazole

Die Erfindung betrifft neue substituierte 5-Ethylamino-1-arylpyrazole, mehrere Verfahren zu ihrer Herstellung und ihre Verwendung als Schädlingsbekämpfungsmittel.

Es ist bereits bekannt, daß bestimmte 5-Alkylamino-1-arylpyrazole wie beispielsweise das 1-(2,6-Dichlor-4-trifluormethyl-phenyl)-5-methylamino-4-trifluormethylthiopyrazol oder das 1-(2,6-Dichlor-4-trifluormethyl-phenyl)-5-methylamino-4-dichlorfluormethylthiopyrazol insektizide, akarizide und nematizide Eigenschaften besitzen (vgl. EP 201 852).

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Es wurden neue substituierte 5-Ethylamino-1-arylpyrazole der allgemeinen Formel (I),

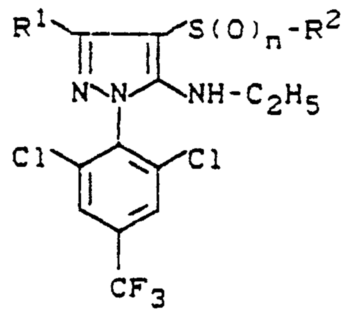

(I)

in welcher
$R^1$ für Wasserstoff oder Alkyl stelt,
$R^2$ für Halogenalkyl steht und
n für eine Zahl 0, 1 oder 2 steht,
mit Ausnahme der Verbindung der Formel (I), bei welcher $R^1$ für Wasserstoff, $R^2$ für Dichlorfluormethyl und gleichzeitig n für 0 steht,
gefunden.

Weiterhin wurde gefunden, daß man die neuen substituierten 5-Ethylamino-1-arylpyrazole der allgemeinen Formel (I),

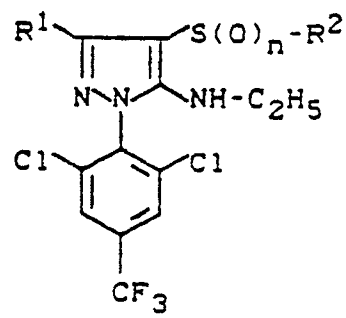

(I)

in welcher
$R^1$ für Wasserstoff oder Alkyl steht,
$R^2$ für Halogenalkyl steht und
n für eine Zahl 0, 1 oder 2 steht,
mit Ausnahme der Verbindung der Formel (I), bei welcher $R^1$ für Wasserstoff, $R^2$ für Dichlorfluormethyl und gleichzeitig n für 0 steht, nach einem der im Folgenden beschriebenen Verfahren erhält:

(a) man erhält 1-Arylpyrazole der Formel (I),

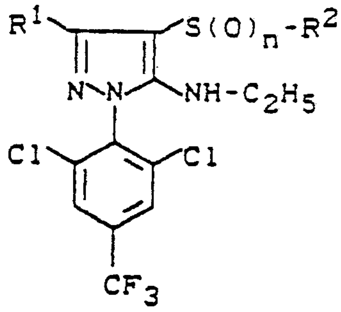

(I)

in welcher
$R^1$, $R^2$ und n die oben angegebene Bedeutung haben,
wenn man 5-Amino-1-aryl-pyrazole der Formel (II),

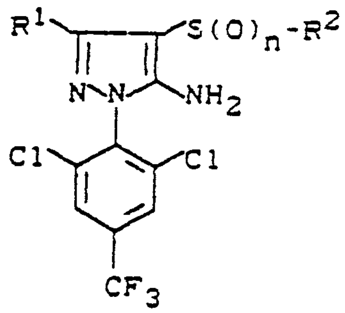

(II)

in welcher
$R^1$, $R^2$ und n die oben angegebene Bedeutung haben,
mit Alkylierungsmitteln der Formel (III),

$C_2H_5$-E (III)

in welcher
E für eine elektronenanziehende Abgangsgruppe steht,
gegebenenfalls in Gegenwart eines Verdünnungsmittels und gegebenenfalls in Gegenwart eines Säurebindemittels sowie gegebenenfalls in Gegenwart eines Katalysators umsetzt;

(b) man erhält substituierte 1-Arylpyrazole der Formel (I),

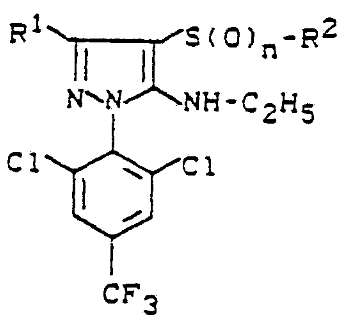

(I)

in welcher
$R^1$, $R^2$ und n die oben angegebene Bedeutung haben,
alternativ auch, wenn man 5-(N-Acylamino)-1-arylpyrazole der Formel (IV),

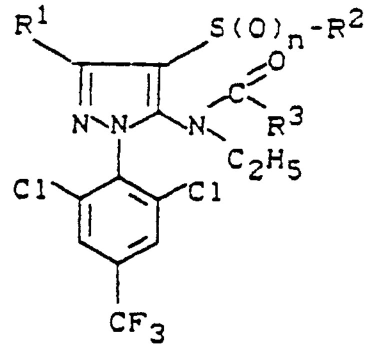

(IV)

in welcher
$R^1$, $R^2$ und n die oben angegebene Bedeutung haben und
$R^3$ für Alkyl steht,
mit Säuren als Katalysator gegebenenfalls in Gegenwart eines Verdünnungsmittels deacyliert;

(c) man erhält substituierte 1-Arylpyrazole der Formel (Ia),

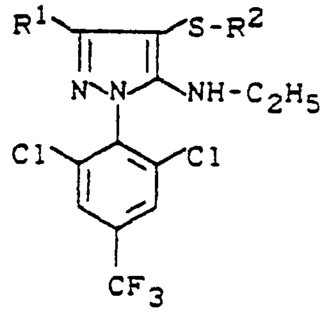

(Ia)

in welcher
$R^1$ und $R^2$ die oben angegebene Bedeutung haben,
alternativ auch, wenn man 4-unsubstituierte 1-Arylpyrazole der Formel (V),

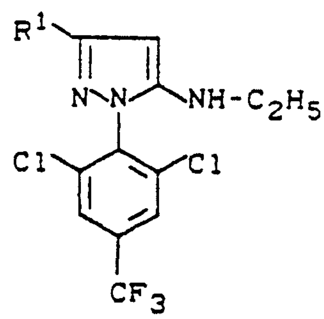

(V)

in welcher
$R^1$ die oben angegebene Bedeutung hat,
mit Sulfenylhalogeniden der Formel (VI),

$R^2$-S-$Hal^1$ (VI)

in welcher
$R^2$ die oben angegebene Bedeutung hat und
$Hal^1$ für Halogen steht,
gegebenenfalls in Gegenwart eines Verdünnungsmittels und gegebenenfalls in Gegenwart eines Säurebindemittels umsetzt;

(d) man erhält 1-Arylpyrazole der Formel (Ib),

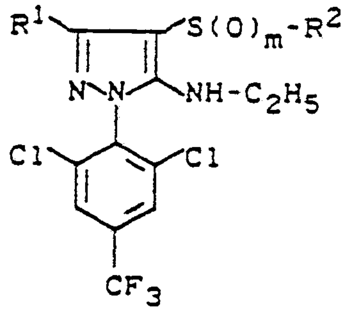

(Ib)

in welcher
R' und R² die oben angegebene Bedeutung haben und
m für eine Zahl 1 oder 2 steht,
alternativ auch, wenn man 1-Aryl-pyrazole der Formel (Ia),

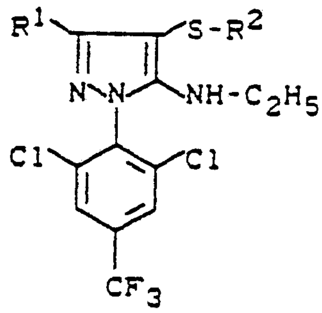

(Ia)

in welcher
R' und R² die oben angegebene Bedeutung haben,
mit einem Oxidationsmittel, gegebenenfalls in Gegenwart eines Verdünnungsmittels und gegebenenfalls in Gegenwart eines Katalysators sowie gegebenenfalls in Gegenwart eines Säurebindemittels umsetzt;

(e) man erhält 1-Arylpyrazole der Formel (Ic),

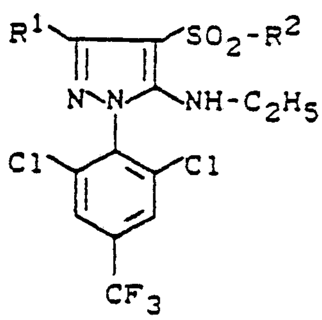

(Ic)

in welcher
R' und R² die oben angegebene Bedeutung haben,
alternativ auch, wenn man 5-Halogen-1-arylpyrazole der Formel (VII),

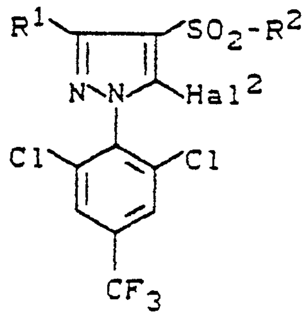

(VII)

in welcher
$R^1$ und $R^2$ die oben angegebene Bedeutung haben und
$Hal^2$ für Halogen steht,
mit Ethylamin der Formel (VIII),

$C_2H_5$-$NH_2$ (VIII)

gegebenenfalls in Gegenwart eines Verdünnungsmittels und gegebenenfalls in Gegenwart eines Säurebindemittels umsetzt.

Schließlich wurde gefunden, daß die neuen substituierten 5-Ethylamino-1-arylpyrazole der Formel (I) eine sehr gute Wirkung gegen tierische Schädlinge besitzen.

Überraschenderweise zeigen die erfindungsgemäßen substituierten 5-Ethylamino-1-arylpyrazole eine erheblich bessere Wirkung gegen tierische Schädlinge als die aus dem Stand der Technik bekannten 1-Arylpyrazole, wie beispieslweise das 1-(2,6-Dichlor-4-trifluormethylphenyl)-5-methylamino-4-dichlorfluormethylthiopyrazol oder das 1-(2,6-Dichlor-4-trifluormethylphenyl)-5-methylamino-4-trifluormethylthio-pyrazol, welche chemisch und wirkungsmäßig naheliegende Verbindungen sind.

Die erfindungsgemäßen substituierten 5-Ethylamino-1-arylpyrazole sind durch die Formel (I) allgemein definiert. Bevorzugt sind Verbindungen der Formel (I), bei welchen
$R^1$ für Wasserstoff oder für geradkettiges oder verzweigtes Alkyl mit 1 bis 4 Kohlenstoffatomen steht,
$R^2$ für geradkettiges oder verzweigtes Halogenalkyl mit 1 bis 4 Kohlenstoffatomen und 1 bis 9 gleichen oder verschiedenen Halogenatomen steht und
n für eine Zahl 0, 1 oder 2 steht,
mit Ausnahme der Verbindung der Formel (I), bei welcher $R^1$ für Wasserstoff, $R^2$ für Dichlorfluormethyl und gleichzeitig n für 0 steht.

Besonders bevorzugt sind Verbindungen der Formel (I), bei welchen
$R^1$ für Wasserstoff, Methyl oder Ethyl steht,
$R^2$ für Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl , Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl oder Fluorchlorbrommethyl steht und
n für eine Zahl 0, 1 oder 2 steht,
mit Ausnahme der Verbindungen der Formel (I), bei welcher $R^1$ für Wasserstoff, $R^2$ für Dichlorfluormethyl und gleichzeitig n für 0 steht.

Ganz besonders bevorzugt sind Verbindungen der Formel (I), bei welcher
$R^1$ für Wasserstoff oder Methyl steht,
$R^2$ für Trifluormethyl, Dichlorfluormethyl oder Difluorchlormethyl steht und
n für eine Zahl 0, 1 oder 2 steht,
mit Ausnahme der Verbindung der Formel (I), bei welcher $R^1$ für Wasserstoff, $R^2$ für Dichlorfluormethyl und gleichzeitig n für 0 steht.

Verwendet man beispielsweise 5-Amino-4-trifluormethylthio-1-(2,6-dichlor-4-trifluormethylphenyl)-pyrazol und Ethyliodid als Ausgangsstoffe, so läßt sich der Reaktionsablauf des erfindungsgemäßen Verfahrens (a) durch das folgende Formelschema darstellen:

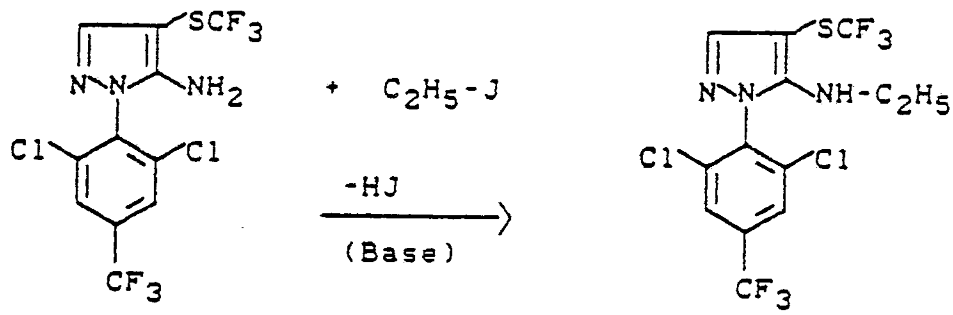


Verwendet man beispielsweise 3-Methyl-4-trifluormethylthio-5-(N-ethyl-acetamido)-1-(2,6-dichlor-4-trifluormethylphenyl)-pyrazol als Ausgangsverbindung, so läßt sich der Reaktionsablauf des erfindungsgemäßen Verfahrens (b) durch das folgende Formelschema darstellen:

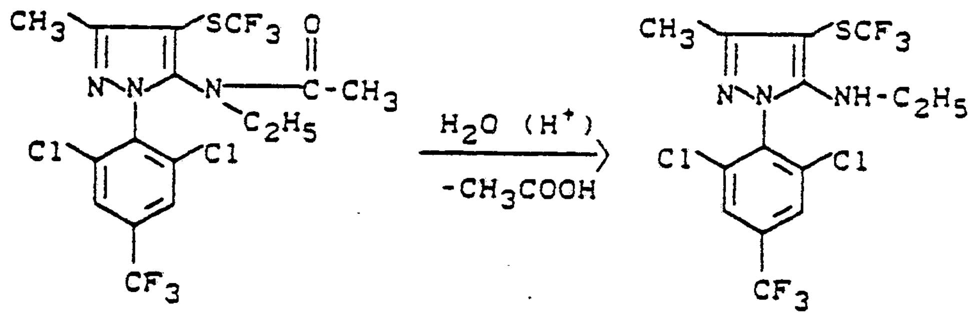


Verwendet man beispielsweise 3-Methyl-1-(2,6-dichlor-4-trifluormethylphenyl)-5-ethylamino-pyrazol und Trifluormethansulfenylchlorid als Ausgangsstoffe, so läßt sich der Reaktionsablauf des erfindungsgemäßen Verfahrens (c) durch das folgende Formelschema darstellen:

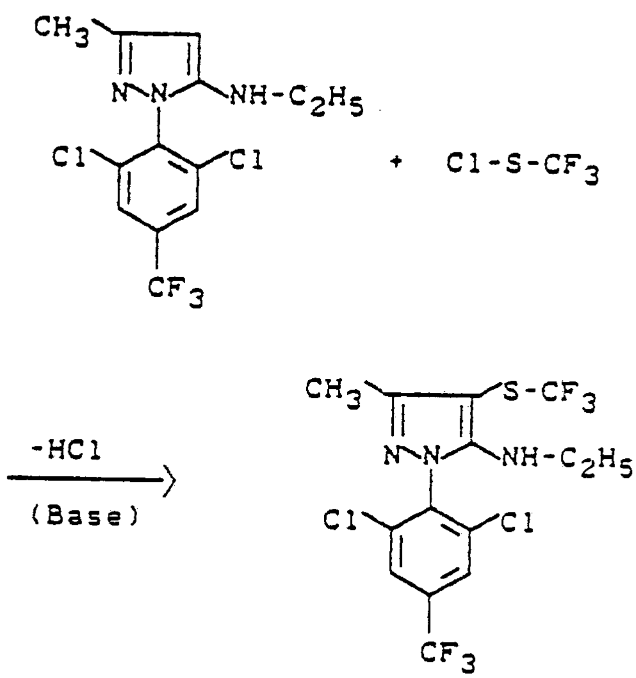


Verwendet man beispielsweise 5-Ethylamino-4-trifluormethylthio-1-(2,6-dichlor-4-trifluormethylphenyl)-pyrazol als Ausgangsverbindung und m-Chlorperbenzoesäure als Oxidationsmittel, so läßt sich der Reaktionsablauf des erfindungsgemäßen Verfahrens (d) durch das folgende Formelschema darstellen:

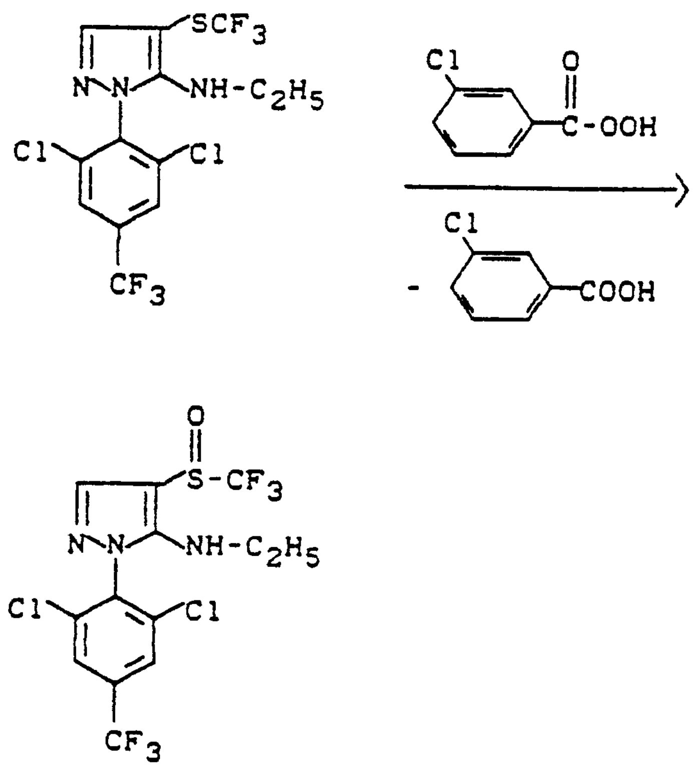

Verwendet man beispielsweise 4-Dichlorfluormethylsulfonyl-5-brom-1-(2,6-dichlor-4-trifluormethyl-phenyl)-pyrazol und Ethylamin als Ausgangsstoffe, so läßt sich der Reaktionsablauf des erfindungsgemäßen Verfahrens (e) durch das folgende Formelschema darstellen:

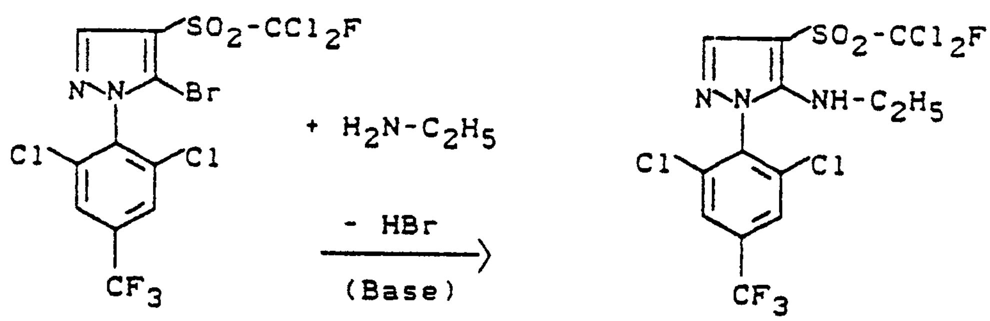


Die zur Durchführung des erfindungsgemäßen Verfahrens (a) als Ausgangsstoffe benötigten 5-Amino-1-arylpyrazole sind durch die Formel (II) allgemein definiert. In dieser Formel (II) stehen $R^1$, $R^2$ und n vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

Die Amino-1-aryl-pyrazole der Formel (II) sind bekannt (vgl. EP 201 852).

Die zur Durchführung des erfindungsgemäßen Verfahrens (a) weiterhin als Ausgangsstoffe benötigten Alkylierungsmittel sind durch die Formel (III) allgemein definiert. In dieser Formel (III) steht E vorzugsweise für Halogen, insbesondere für Chlor, Brom oder Iod oder für Ethoxysulfonyloxy oder p-Toluolsulfonyloxy.

Die Alkylierungsmittel der Formel (III) sind allgemein bekannte Verbindungen der organischen Chemie.

Die zur Durchführung des erfindungsgemäßen Verfahrens (b) als Ausgangsstoffe benötigten 5-(N-Acyl-amino)-1-arylpyrazole sind durch die Formel (IV) allgemein definiert. In dieser Formel (IV) stehen $R^1$, $R^2$ und n vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

$R^3$ steht vorzugsweise für geradkettiges oder verzweigtes Alkyl mit 1 bis 4 Kohlenstoffatomen, insbesondere für Methyl oder Ethyl.

Die N-Acylamino-1-aryl-pyrazole der Formel (IV) sind noch nicht bekannt. Man erhält sie jedoch in Analogie zu bekannten Verfahren (vgl. z.B. EP 201 852), wenn man 5-(N-Acylamino)-pyrazole der Formel

(IX),

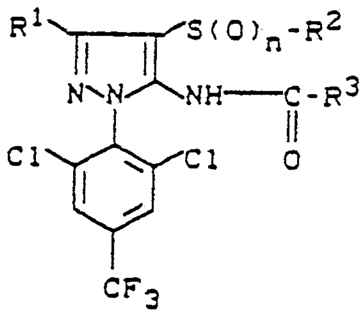

(IX)

in welcher
$R^1$, $R^2$, $R^3$ und n die oben angegebene Bedeutung haben,
mit Alkylierungsmitteln der Formel (III),

$C_2H_5$-E (III)

in welcher
E für eine elektronenanziehende Abgangsgruppe, wie beispielsweise Halogen, insbesondere für Chlor, Brom oder lod oder für Ethoxysulfonyloxy oder p-Toluolsulfonyloxy steht,
in Analogie zur Durchführung des erfindungsgemäßen Verfahrens (a) gegebenenfalls in Gegenwart eines Verdünnungsmittels wie beispielsweise Dichlormethan und gegebenenfalls in Gegenwart einer Base wie beispielweise Natronlauge sowie gegebenenfalls in Gegenwart eines Phasentransferkatalysators wie beispielsweise Tributylbenzylammoniumchlorid bei Temperaturen zwischen 0 °C und 120 °C umsetzt.

Die 5-(N-Acylamino)-pyrazole der Formel (IX) sind bekannt (vgl. EP 201 852).

Die zur Durchführung des erfindungsgemäßen Verfahrens (c) als Ausgangsstoffe benötigten 4-unsubstituierten1-Arylpyrazole sind durch die Formel (V) allgemein definiert. In dieser Formel (V) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden.

Die 4-unsubstituierten 1-Arylpyrazole der Formel (V) sind noch nicht bekannt.

Man erhält sie jedoch in Analogie zu bekannten Verfahren (vgl. EP 201 852), wenn man 5-Amino-1-arylpyrazole der Formel (X),

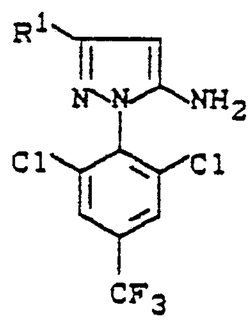

(X)

in welcher
$R^1$ die oben angegebene Bedeutung hat,
mit Alkylierungsmitteln der Formel (III),

$C_2H_5$-E (III)

in welcher
E für eine elektronenanziehende Abgangsgruppe, wie beispielsweise Halogen, insbesondere für Chlor, Brom oder lod oder für Ethoxysulfonyloxy oder p-Toluolsulfonyloxy steht,
in Analogie zur Durchführung des erfindungsgemäßen Verfahren (a) gegebenenfalls in Gegenwart eines Verdünnungsmittels wie beispielsweise Dichlormethan bei Temperaturen zwischen 0 °C und 150 °C umsetzt; in einer Reaktionsvariante zu diesem Verfahren ist es auch möglich das Alkylierungsmittel der Formel (III) für den Fall, daß E in Formel (III) für eine Ethoxysulfonyloxygruppe steht direkt im Reaktionsgefäß aus konzentrierter Schwefelsäure und Ethanol herzustellen und im Eintopfverfahren mit den 5-Amino-1-aryl-pyrazolen der Formel (X) direkt weiter umzusetzen (vgl. auch die Herstellungsbeispiele).

Die 5-Amino-1-aryl-pyrazole der Formel (X) sind bekannt oder erhältlich in Analogie zu bekannten Verfahren (vgl. EP 201 852).

Die zur Durchführung des erfindungsgemäßen Verfahrens (c) weiterhin als Ausgangsstoffe benötigten Sulfenylhalogenide sind durch die Formel (VI) allgemein definiert. In dieser Formel (VI) steht $R^2$ vorzugsweise für diejenigen Reste, die bereits im Zusammenhang mit der Beschreibung der erfindungsgemäßen Stoffe der Formel (I) als bevorzugt für diesen Substituenten genannt wurden.

$Hal^1$ steht vorzugsweise für Chlor.

Die Sulfenylhalogenide der Formel (VI) sind allgemein bekannte Verbindungen der organischen Chemie.

Die zur Durchführung des erfindungsgemäßen Verfahrens (d) als Ausgangsstoffe benötigten 1-Arylpyrazole sind durch die Formel (Ia) allgemein definiert. In dieser Formel (Ia) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Arylpyrazole der Formel (Ia) sind erfindungsgemäße Verbindungen und erhältlich mit Hilfe der erfindungsgemäßen Verfahren (a), (b) oder (c).

Die zur Durchführung des erfindungsgemäßen Verfahrens (e) als Ausgangsstoffe benötigten 5-Halogen-1-aryl-pyrazole sind durch die Formel (VII) allgemein definiert. In dieser Formel (VII) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

$Hal^2$ steht vorzugsweise für Chlor oder Brom.

Die 5-Halogen-1-aryl-pyrazole der Formel (VII) sind bekannt (vgl. DE-OS 3 529 829).

Das zur Durchführung des erfindungsgemäßen Verfahrens (e) weiterhin als Ausgangsverbindung benötigte Ethylamin ist eine allgemein bekannte Verbindung der organischen Chemie.

Als Verdünnungsmittel zur Durchführung des erfindungsgemäßen Verfahrens (a) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylen glykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-dimethylammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Säurebindemittel zur Durchführung des erfindungsgemäßen Verfahrens (a) kommen alle üblicherweise verwendbaren anorganischen und organischen Basen infrage. Vorzugsweise verwendet man Alkalimetallhydride, -hydroxide, -amide, -alkholate, -carbonate oder hydrogencarbonate, wie beispielsweise Natriumhydrid, Natriumamid, Natriummethylat, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (a) setzt man pro Mol 5-Amino-1-aryl-pyrazol der Formel (II) im allgemeinen 1.0 bis 20.0 Mol, vorzugsweise 1.0 bis 15.0 Mol an Alkylierungsmittel der Formel (III) und gegebenenfalls 1.0 bis 3.0 Mol, vorzugsweise 1.0 bis 2.0 Mol an Säurebindemittel sowie 0.01 bis 1.0 Mol an Phasentransferkatalysator ein. Die Reaktionsdurchführung, Aufarbeitung und Isolierung der Reaktionsprodukte der Formel (I) erfolgt in allgemein üblicher Art und Weise.

Als Verdünnungsmittel zur Durchführung des erfindungsgemäßen Verfahrens (b) kommen anorganische oder organische polare Lösungsmittel in Frage. Vorzugsweise verwendet man Alkohole, wie beispielsweise Methanol, Ethanol oder Propanol, oder deren Gemische mit Wasser.

Als Katalysatoren zur Durchführung des erfindungsgemäßen Verfahrens (b) kommen vorzugsweise anorganische Mineralsäuren, insbesondere Chlorwasserstoffsäure oder Schwefelsäure infrage.

Die Reaktionstemperaturen können bei der Durchführung des erfindungsgemäßen Verfahrens (b) in einem größeren Bereich variiert werden. Im allgemeinen arbeitet man zwischen +20 °C und +150 °C,

vorzugsweise zwischen +50 °C und +120 °C.

Zur Durchführung des erfindungsgemäßen Verfahrens (b) setzt man pro Mol 5-(N-Acylamino)-1-aryl-pyrazol der Formel (IV) im allgemeinen 1,0 bis 20,0 Mol, vorzugsweise 1,0 bis 10,0 Mol an Katalysatorsäure ein und erwärmt für mehrere Stunden auf die erforderliche Reaktionstemperatur. Die Aufarbeitung, Isolierung und Reinigung der Reaktionsprodukte der Formel (I) erfolgt nach üblichen Methoden.

Als Verdünnungsmittel zur Durchführung des erfindungsgemäßen Verfahrens (c) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Säuren wie beispielsweise Essigsäure.

Das erfindungsgemäße Verfahren (c) wird gegebenenfalls in Gegenwart eines Säurebindemittels durchgeführt. Als solche kommen alle üblichen anorganischen oder organischen Base infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen(DBU).

Die Reaktionstemperaturen können bei der Durchführung des erfindungsgemäßen Verfahrens (c) in einem größeren Bereich variiert werden. Im allgemeinen arbeitet man bei Temperaturen zwischen -20 °C und +120 °C, vorzugsweise bei Temperaturen zwischen 0 °C und + 50 °C.

Zur Durchführung des erfindungsgemäßen Verfahrens (c) setzt man pro Mol an 4-unsubstituierten 1-Aryl-pyrazol der Formel (V) im allgemeinen 1.0 bis 2.5 Mol, vorzugsweise 1.0 bis 1.5 Mol an Sulfenylhalogenid der Formel (VI) und 1.0 bis 2.5 Mol, vorzugsweise 1.0 bis 1.5 Mol an Säurebindemittel ein. Die Reaktionsführung, Aufarbeitung und Isolierung der Reaktionsprodukte der Formel (Ia) erfolgt nach allgemein üblichen Verfahren.

Als Oxidationsmittel zur Durchführung des erfindungsgemäßen Verfahrens (d) kann man alle üblicherweise für Schwefeloxidationen infrage kommenden anorganischen oder organischen Oxidationsmittel verwenden. Vorzugsweise verwendet man organische Persäuren, wie beispielsweise Peressigsäure, 4-Nitroperbenzoesäure oder 3-Chlorperbenzoesäure, anorganische Persäuren, wie beispielsweise Periodsäure oder auch Wasserstoffperoxid, Kaliumpermanganat oder Chromsäure.

Als Verdünnungsmittel zur Durchführung des erfindungsgemäßen Verfahrens (d) kommen ebenfalls inerte organische Lösungsmittel infrage.

Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure, oder dipolar aprotische Lösungsmittel, wie Acetonitril, Aceton, Essigsäureethylester oder Dimethylformamid.

Das erfindungsgemäße Verfahren (d) kann gegebenenfalls in Gegenwart eines Säurebindemittels durchgeführt werden. Als solche kommen alle üblicherweise verwendbaren organischen und anorganischen Säurebindemittel infrage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Das erfindungsgemäße Verfahren (d) kann gegebenenfalls in Gegenwart eines geeigneten Katalysators durchgeführt werden. Als solche kommen alle üblicherweise für derartige Schwefeloxidationen üblichen Katalysatoren infrage. Beispielhaft genannt seien in diesem Zusammenhang Schwermetallkatalysatoren wie Ammoniummolybdat.

Die Reaktionstemperaturen können bei der Durchführung des erfindungsgemäßen Verfahrens (d) in einem größeren Bereich variiert werden. In allgemeinen arbeitet man bei Temperaturen zwischen -20 °C und +70 °C, vorzugsweise bei Temperaturen zwischen 0 °C und +50 °C.

Zur Durchführung des erfindungsgemäßen Verfahrens (d) setzt man pro Mol an 1-Aryl-pyrazol der Formel (Ia) im allgemeinen 0.8 bis 1.2 Mol, vorzugsweise äquimolare Mengen Oxidationsmittel ein, wenn man die Oxidation des Schwefels auf der Sulfoxidstufe unterbrechen will. Zur Oxidation zum Sulfon setzt man pro Mol an 1-Aryl-pyrazol der Formel (Ia) im allgemeinen 1.8 bis 3.0 Mol, vorzugsweise doppelt molare Mengen an Oxidationsmittel ein. Die Reaktionsführung, Aufarbeitung und Isolierung der Endprodukte der Formel (Ib) erfolgt nach üblichen Verfahren.

Als Verdünnungsmittel zur Durchführung des erfindungsgemäßen Verfahrens (e) kommen inerte organische Lösungsmittel in Frage. Hierzu gehören insbesondere aliphatische, alicyclische oder aromatische,

gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetra chlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide wie Dimethylsulfoxid.

Das erfindungsgemäße Verfahren (e) kann gegebenenfalls in Gegenwart eines geeigneten Säurebindemittels durchgeführt werden. Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).
Es ist jedoch auch möglich, einen entsprechenden Überschuß an dem als Reaktionspartner eingesetzten Ethylamin der Formel (VIII) gleichzeitig als Säurebindemittel zu verwenden.

Die Reaktionstemperaturen können bei der Durchführung des erfindungsgemäßen Verfahrens (e) in einem größeren Bereich variiert werden. Im allgemeinen arbeitet man bei Temperaturen zwischen -20 °C und +200 °C, vorzugsweise bei Temperaturen zwischen 0 °C und +150 °C.

Zur Durchführung des erfindungsgemäßen Verfahrens (e) setzt man pro Mol an 5-Halogen-1-arylpyrazol der Formel (VII) im allgemeinen 1.0 bis 10.0 Mol, vorzugsweise 1.0 bis 5.0 Mol an Ethylamin der Formel (VIII) ein. Die Reaktionsdurchführung, Aufarbeitung und Isolierung der Reaktionsprodukte der Formel (Ic) erfolgt nach allgemein üblichen Verfahren.

Die Wirkstoffe eignen sich bei guter Pflanzenverträglichkeit und günstiger Warmblütertoxizität zur Bekämpfung von tierischen Schädlingen, insbesondere Insekten und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorr hoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus,

Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räubemilben, Laufmilben, Fliegen (stechend und leckend), parasitierende Fliegenlarven, Läuse, Haarlinge, Federlinge, Flöhe.

Sie sind gegen normalsensible und resistente Arten und Stämme, sowie gegen alle parasitierenden und nicht parasitierenden Entwicklungsstadien der Ektoparasiten und Endoparasiten wirksam.

Die erfindungsgemäßen Wirkstoffe der Formel (I) zeichnen sich durch eine hohe insektizide Wirksamkeit aus. Sie lassen sich insbesondere gegen pflanzenschädigende Insekten, wie beispielsweise gegen die gemeine Bohnenblattlaus (Aphis fabae) oder gegen die Larven der Meerrettichblattkäfer (Phaedon cochleariae), einsetzen. Dabei zeigen die erfindungsgemäßen Wirkstoffe auch blattsystemische Eigenschaften. Sie eignen sich daneben hervorragend zur Bekämpfung von Bodeninsekten und lassen sich beispielsweise zur Bekämpfung von Phorbiase antiqua-Maden oder Diabrotica balteata-Larven im Boden einsetzen. Auch eine nennenswerte wurzelsystemische Wirkung, beispielsweise gegen Phaedon cochleariae-Larven ist hervorzuheben.

Außerdem besitzen die erfindungsgemäßen Wirkstoffe der Formel (I) eine hohe Wirkung gegen Hygiene- und Vorratsschädlinge und lassen sich beispielsweise zur Bekämpfung der gemeinen Stubenfliege (Musca domestica) oder zur Bekämpfung von Mückenlarven (Aedes aegypti) einsetzen.

Darüber hinaus lassen sich die erfindungsgemäßen Wirkstoffe mit besonders gutem Erfolg zur Bekämpfung von von parasitisch lebenden Warmblüterschädlingen, wie beispielsweise gegen die Larven der Goldfliege (Lucilia cuprina) oder gegen Rinderzecken (Boophilus microplus) sowie gegen endoparasitisch lebende Nematoden der Gattung Caenorhabditis elegans einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Träger stoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse

Kieselsäure. Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegatabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Die biologische Wirksamkeit der erfindungsgemäßen Verbindungen soll anhand der folgenden Beispiele erläutert werden.

Herstellungsbeispiel

Beispiel 1

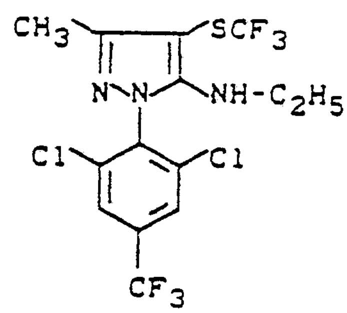

(Verfahren c)

In eine Lösung aus 10,1 g (0,03 Mol) 5-Ethylamino-3-methyl-1-(2,6-dichlor-4-trifluormethylphenyl)-pyrazol in 50 ml Eisessig leitet man bei 15 °C bis 20 °C 4,2 g (0,033 Mol) Trifluormethansulfenylchlorid, rührt 6 Stunden bei Raumtemperatur, gießt die Mischung in 1 l Eiswasser, rührt eine weitere Stunde, saugt den ausgefallenen Niederschlag ab, wäscht ihn mit Wasser neutral und trocknet ihn.

Man erhält so 10,4 g (79 % der Theorie) an 5-Ethylamino-3-methyl-4-trifluormethylthio-1-(2,6-dichlor-4-trifluormethylphenyl)-pyrazol vom Schmelzpunkt 53 °C -54 °C.

Herstellung der Ausgangsverbindung

Beispiel V-1

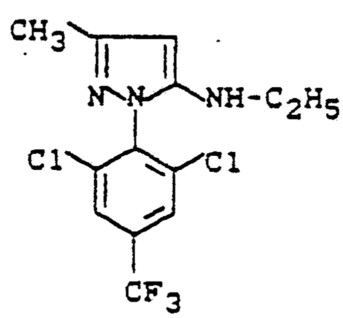

Zu 15,5 g (0,05 Mol) 5-Amino-3-methyl-1-(2,6-dichlor-4-trifluormethylphenyl)-pyrazol (vgl. EP 201 852) in 30 ml konzentrierter Schwefelsäure tropft man bei Raumtemperatur 15 ml (11,9 g; 0.26 Mol) Ethanol, rührt nach beendeter Zugabe 2 Stunden bei 110 °C (bis im Dünnschichtchromatogramm kein Ausgangsprodukt mehr nachweisbar ist), gibt die Reaktionsmischung in 300 ml Eiswasser, extrahiert zweimal mit jeweils 100 ml Hexan, stellt die wässrige Phase mit Natronlauge auf pH 7 ein, rührt 20 Stunden bei Raumtemperatur, saugt den kristallinen Niederschlag ab, wäscht mit Wasser und trocknet.

Man erhält 11 g (65 % der Theorie) an 5-Ethylamino-3-methyl-1-(2,6-dichlor-4-trifluormethylphenyl)-pyrazol vom Schmelzpunkt 74 °C - 75 °C.

Beispiel 2

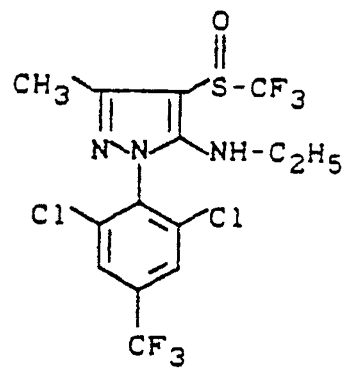

(Verfahren d)

Zu 3 g (0,007 Mol) 5-Ethylamino-3-methyl-4-trifluormethylthio-1-(2,6-dichlor-4-trifluormethylphenyl)-pyrazol in 30 ml Schwefelsäure gibt man bei Raumtemperatur 0,7 g (0,007 Mol) einer 35prozentigen wässrigen Wasserstoffperoxidlösung, rührt anschließend 20 Stunden bei Raumtemperatur, gießt die Mischung in Eiswasser, extrahiert mehrfach mit Dichlormethan, trocknet die vereinigten organischen Phasen über Magnesiumsulfat und entfernt das Lösungsmittel im Vakuum.

Man erhält 1,6 g (50 % der Theorie) an 5-Ethylamino-3-methyl-4-trifluormethylsulfinyl-1-(2,6-dichlor-4-trifluormethylphenyl)-pyrazol vom Schmelzpunkt 76 °C.

In entsprechender Weise und gemäß den allgemeinen Angaben zur Herstellung erhält man die folgenden 1-Aryl-pyrazole der allgemeinen Formel (I):

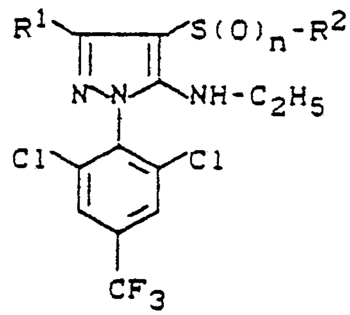

(I)

| Bsp. Nr. | $R^1$ | $R^2$ | n | Schmelzpunkt /°C |
|---|---|---|---|---|
| 3 | $CH_3$ | $-CCl_2F$ | 0 | 77-81 |
| 4 | $CH_3$ | $-CF_3$ | 2 | 107-108 |
| 5 | $CH_3$ | $-CCl_2F$ | 1 | 63-65 |
| 6 | $CH_3$ | $-CCl_2F$ | 2 | 160-162 |
| 7 | H | $-CF_3$ | 0 | 57-58 |
| 8 | H | $-CCl_2F$ | 1 | 53-58 |
| 9 | H | $-CCl_2F$ | 2 | 99-102 |
| 10 | H | $-CF_3$ | 1 | 119-121 |
| 11 | H | $-CF_3$ | 2 | 75-78 |

Anwendungsbeispiele

In den folgenden Anwendungsbeispielen wurden die nachstehend aufgeführten Verbindungen als Vergleichsubstanzen eingesetzt:

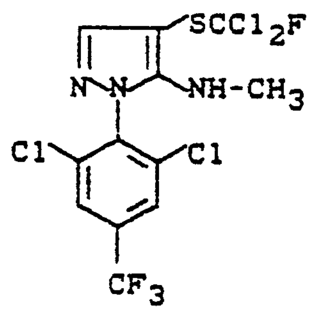

(A)

1-(2,6-Dichlor-4-trifluormethylphenyl)-5-methylamino-4-dichlorfluormethylthio-pyrazol

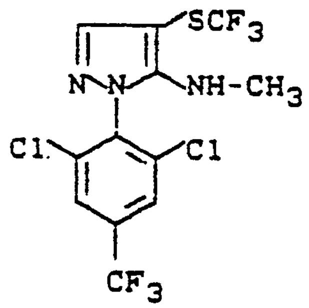

(B)

1-(2,6-Dichlor-4-trifluormethylphenyl)-5-methylamino-4-trifluormethylthio-pyrazol

1-(2,6-Dichlor-4-trifluormethylphenyl)-5-ethylamino-4-difluormethylthio-pyrazol

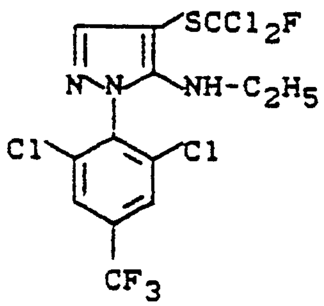

(C)

(Alle bekannt aus EP 201 852)

Beispiel A

Mückenlarven-Test

Testtiere: 4. Larvenstadium Aedes aegypti
Lösungsmittel: 99 Gewichtsteile Aceton
Emulgator: 1 Gewichtsteil Benzylhydroxydiphenylpolyglykolether

Zur Herstellung einer zweckmäßigen Wirkstoffzubereitung löst man 2 Gew.-Teile Wirkstoff in 1000 Volumenteilen Lösungsmittel, das Emulgator in der oben angegebenen Menge enthält. Die so erhaltene Lösung wird mit Wasser auf die gewünschten geringeren Konzentrationen verdünnt.

Man füllt die wäßrigen Wirkstoffzubereitungen der gewünschten Konzentration in Kunststoffbecher und setzt ansschließend 25 Mückenlarven in jeden Becher ein. Die Larven werden täglich mit Fischfutter (Tetramin®) gefüttert.

Nach 24 Stunden wird die Abtötung in % bestimmt. Dabei bedeutet 100 %, daß alle Larven abgetötet worden sind. 0 % bedeutet, daß überhaupt keine Larven abgetötet worden sind.

Bei diesem Test zeigen z.B. die folgenden Verbindungen der Herstellungsbeispiele überlegene Wirkung gegenüber dem Stand der Technik: 1, 2, 3, 4, 7, 9, 11.

Beispiel B

$LT_{100}$-Test für Dipteren

Testtiere: Musca domestica, resistent
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: 1, 2, 4, 7, 10, 11.

Beispiel C

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewübschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 4, 8, 9.

Beispiel D

Aphis-Test (systemische Wirkung)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Aphis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 1, 2, 7, 9,.10, 11.

## Beispiel E

Grenzkonzentrations-Test / Wurzelsystemische Wirkung

Testinsekt: Phaedon cochleariae-Larven
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 7, 9, 11.

## Beispiel F

Grenzkonzentrations-Test ' Bodeninsekten

Testinsekt: Phorbia antiqua-Maden (im Boden)
Lösungsmittel: 3 Gewichtsteile Aceton
Emulgator: 1 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt

diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 4, 8.

## Beispiel G

Grenzkonzentrations-Test / Bodeninsekten

Testinsekt: Diabrotica balteata-Larven (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 4.

## Beispiel H

Test mit Lucilia cuprina resistent-Larven

Emulgator:
35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 7, 8, 9, 10, 11.

## Beispiel I

Test mit Boophilus microplus resistent

Lösungsmittel:
35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 1,2.

Beispiel J

In vitro Nematodentest

Caenorhabditis elegans

$10^{-4}$ g Wirkstoff werden in 1 ml Wasser oder 0,1 ml Dimethylsulfoxid (DMSO) gelöst. Diese Lösung wird auf eine Replica-Platte gegeben. Dazu gibt man 2 ml einer E.coli-Suspension, zu der man 10 - 20 weibliche Tiere oder Larven von Caenorhabditis elegans in 0,5 ml steriler M9 Pufferlösung gegeben hat. Die E.coli-Suspension wird hergestellt, indem man 300 ml einer Übernachtkultur eines Uracil-bedürftigen E.coli-Stammes mit 1,8 l steriler M9 Pufferlösung versetzt.

Der Versuchsansatz wird 7 Tage bei 22 °C inkubiert und danach ausgewertet. Es wird bewertet, inwieweit der Wirkstoff die Vermehrung beeinträchtigt und die Konzentration angegeben bei der die Vemehrung verhindert wird.

Bei diesem Test zeigen z.B. die folgenden Verbindungen der Herstellungbeispiele eine mindestens 95 %ige Hemmung der Vermehrung des Nematoden C. elegans
- bei einer Konzentration von ≦ 100 µg/ml: 2, 3;
- bei einer Konzentration von ≦ 10µg/ml: 1, 4, 7, 9, 11.

## Ansprüche

1. Substituierte 5-Ethylamino-1-arylpyrazole der allgemeinen Formel (I)

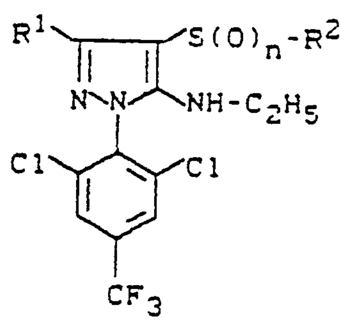

(I)

in welcher
$R^1$ für Wasserstoff oder Alkyl steht,
$R^2$ für Halogenalkyl steht und

n für eine Zahl 0, 1 oder 2 steht,
mit Ausnahme der Verbindung der Formel (I), bei welcher $R^1$ für Wasserstoff, $R^2$ für Dichlorfluormethyl und gleichzeitig n für 0 steht.

2. 5-Ethylamino-1-arylpyrazole gemäß Formel (I) des Anspruchs 1), in welcher
$R^1$ für Wasserstoff oder für geradkettiges oder verzweigtes Alkyl mit 1 bis 4 Kohlenstoffatomen steht,
$R^2$ für geradkettiges oder verzweigtes Halogenalkyl mit 1 bis 4 Kohlenstoffatomen und 1 bis 9 gleichen oder verschiedenen Halogenatomen steht und
n für eine Zahl 0, 1 oder 2 steht,
mit Ausnahme der Verbindung der Formel (I), bei welcher $R^1$ für Wasserstoff, $R^2$ für Dichlorfluormethyl und gleichzeitig n für 0 steht.

3. Substituierte 5-Ethylamino-1-arylpyrazole gemäß Formel (I) des Anspruchs 1), in welcher
$R^1$ für Wasserstoff, Methyl oder Ethyl steht,
$R^2$ für Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl , Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl oder Fluorchlorbrommethyl steht und
n für eine Zahl 0, 1 oder 2 steht,
mit Ausnahme der Verbindungen der Formel (I), bei welcher $R^1$ für Wasserstoff, $R^2$ für Dichlorfluormethyl und gleichzeitig n für 0 steht.

4. Substituierte 5-Ethylamino-1-arylpyrazole gemäß Formel (I) des Anspruchs 1), in welcher
$R^1$ für Wasserstoff oder Methyl steht,
$R^2$ für Trifluormethyl, Dichlorfluormethyl oder Difluorchlormethyl steht und
n für eine Zahl 0, 1 oder 2 steht,
mit Ausnahme der Verbindung der Formel (I), bei welcher $R^1$ für Wasserstoff, $R^2$ für Dichlorfluormethyl und gleichzeitig n für 0 steht.

5. Verfahren zur Herstellung von substituierten 5-Ethylamino-1-arylpyrazolen der allgemeinen Formel (I), .

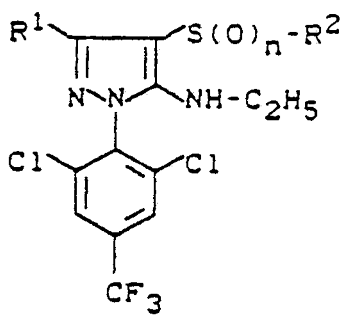

(I)

in welcher
$R^1$ für Wasserstoff oder Alkyl steht,
$R^2$ für Halogenalkyl steht und
n für eine Zahl 0, 1 oder 2 steht,
mit Ausnahme der Verbindung der Formel (I), bei welcher $R^1$ für Wasserstoff, $R^2$ für Dichlorfluormethyl und gleichzeitig n für 0 steht, dadurch gekennzeichnet, daß man zum Erhalt von 1-Arylpyrazolen der Formel (I),

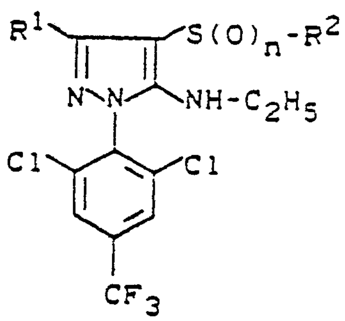

(I)

in welcher
$R^1$, $R^2$ und n die oben angegebene Bedeutung haben,
5-Amino-1-aryl-pyrazole der Formel (II),

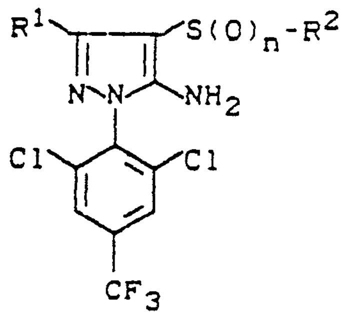

(II)

in welcher
$R^1$, $R^2$ und n die oben angegebene Bedeutung haben,
mit Alkylierungsmitteln der Formel (III),

$C_2H_5$-E (III)

in welcher
E für eine elektronenanziehende Abgangsgruppe steht,
gegebenenfalls in Gegenwart eines Verdünnungsmittels und gegebenenfalls in Gegenwart eines Säurebindemittels sowie gegebenenfalls in Gegenwart eines Katalysators umsetzt;
oder daß man zum Erhalt von substituierten 1-Arylpyrazolen der Formel (I),

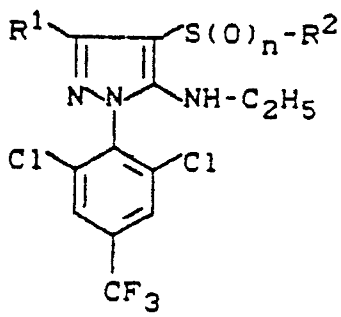

(I)

in welcher
$R^1$, $R^2$ und n die oben angegebene Bedeutung haben,
5-(N-Acylamino)-1-aryl-pyrazole der Formel (IV),

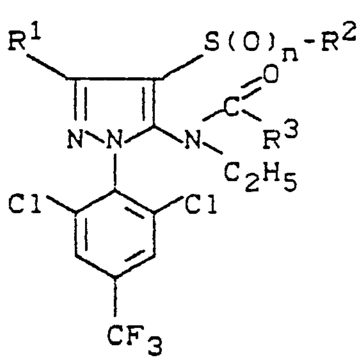

(IV)

in welcher
$R^1$, $R^2$ und n die oben angegebene Bedeutung haben und
$R^3$ für Alkyl steht,
mit Säuren als Katalysator gegebenenfalls in Gegenwart eines Verdünnungsmittels deacyliert;

oder daß man zum Erhalt von substituierten 1-Arylpyrazolen der Formel (Ia),

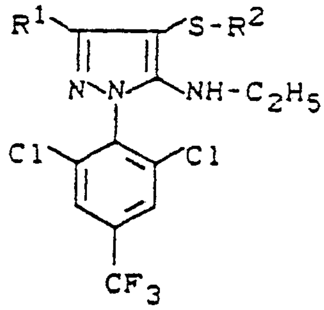

(Ia)

in welcher
R' und $R^2$ die oben angegebene Bedeutung haben,
4-unsubstituierte 1-Arylpyrazole der Formel (V),

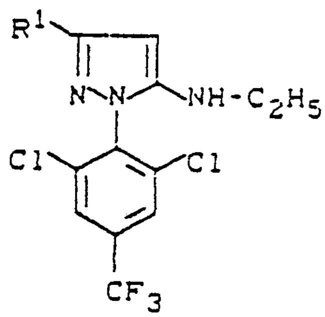

(V)

in welcher
$R^1$ die oben angegebene Bedeutung hat,
mit Sulfenylhalogeniden der Formel (VI),

$R^2$-S-Hal' (VI)

in welcher
$R^2$ die oben angegebene Bedeutung hat und
Hal' für Halogen steht,
gegebenenfalls in Gegenwart eines Verdünnungsmittels und gegebenenfalls in Gegenwart eines Säurebindemittels umsetzt;
oder daß man zum Erhalt von 1-Arylpyrazolen der Formel (Ib),

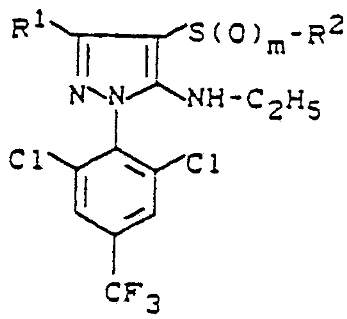

(Ib)

in welcher
$R^1$ und $R^2$ die oben angegebene Bedeutung haben und
m für eine Zahl 1 oder 2 steht,
1-Aryl-pyrazole der Formel (Ia),

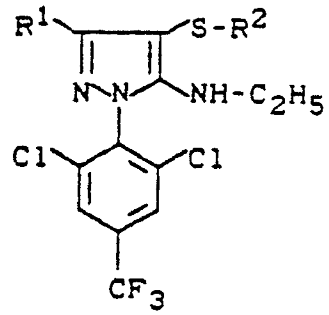

(Ia)

in welcher
$R^1$ und $R^2$ die oben angegebene Bedeutung haben,
mit einem Oxidationsmittel, gegebenenfalls in Gegenwart eines Verdünnungsmittels und gegebenenfalls in Gegenwart eines Katalysators sowie gegebenenfalls in Gegenwart eines Säurebindemittels umsetzt;
oder daß man zum Erhalt von 1-Arylpyrazolen der Formel (Ic),

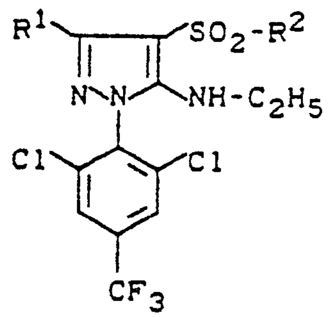

(Ic)

in welcher
$R^1$ und $R^2$ die oben angegebene Bedeutung haben,
5-Halogen-1-arylpyrazole der Formel (VII),

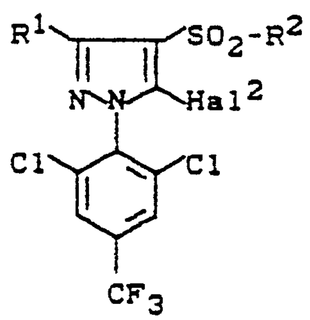

(VII)

in welcher
$R^1$ und $R^2$ die oben angegebene Bedeutung haben und
$Hal^2$ für Halogen steht,
mit Ethylamin der Formel (VIII),

$C_2H_5$-$NH_2$ (VIII)

gegebenenfalls in Gegenwart eines Verdünnungsmittels und gegebenenfalls in Gegenwart eines Säurebindemittels umsetzt.

6. Schädlingsbekämpfungsmittel, gekennzeichnet, durch einen Gehalt an mindestens einem substituierten 5-Ethylamino-1-arylpyrazole der Formel (I).

7. Verfahren zur Bekämpfung von tierischen Schädlingen, dadurch gekennzeichnet, daß man substituierte 5-Ethylamino-1-arylpyrazole der Formel (I) auf tierische Schädlinge und/oder ihren Lebensraum einwirken läßt.

8. Verwendung von substituierte 1-Arylpyrazolen der Formel (I) zur Bekämpfung von tierischen Schädlingen.

9. Verfahren zur Herstellung von Schädlingsbekämpfungsmitteln, dadurch gekennzeichnet, daß man substituierte 5-Ethylamino-1-arylpyrazole der Formel (I) mit Streckmitteln und/oder oberflächenaktiven Mitteln vermischt.

10. 5-(N-Acylamino)-1-aryl-pyrazole der allgemeinen Formel (IV),

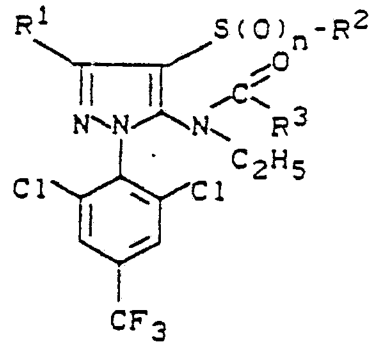

(IV)

in welcher
$R^1$ für Wasserstoff oder Alkyl steht,
$R^2$ für Halogenalkyl steht,
$R^3$ für Alkyl steht und
n für eine Zahl 0,1 oder 2 steht.

11. 5-Ethylamino-1-arylpyrazole der allgemeinen Formel (V),

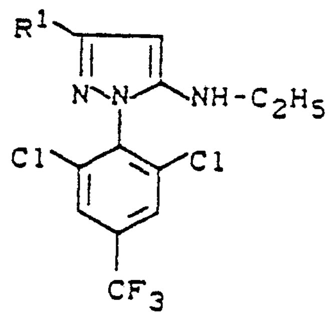

(V)

in welcher
$R^1$ für Wasserstoff oder Alkyl steht.

Europäisches Patentamt

# EUROPÄISCHER RECHERCHENBERICHT

Nummer der Anmeldung

EP 88 10 8602

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | EP-A-0 201 852 (BAYER)<br>* Das ganze Dokument; Seiten 2-9,105 (Nr. 300) * | 1-9 | C 07 D 231/44<br>A 01 N 43/56 |
| P,X | EP-A-0 234 119 (MAY & BAKER)<br>* Das ganze Dokument; Seiten 3-5,7-12 * | 1-4,7-9 | |
| P,A | EP-A-0 260 521 (BAYER)<br>* Das ganze Dokument * | | |
| P,A | EP-A-0 235 628 (BAYER)<br>* Das ganze Dokument * | | |

RECHERCHIERTE SACHGEBIETE (Int. Cl.4)

C 07 D 231/00
A 01 N 43/00

Der vorliegende Recherchenbericht wurde für alle Patentansprüche erstellt

| Recherchenort | Abschlußdatum der Recherche | Prüfer |
|---|---|---|
| DEN HAAG | 18-09-1988 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)